# EUROPÄISCHE PATENTSCHRIFT

(11) **EP 0 614 904 B1**
(45) Veröffentlichungstag und Bekanntmachung des Hinweises auf die Patenterteilung: **02.06.1999**
(21) Anmeldenummer: 94101858.2
(22) Anmeldetag: 08.02.1994
(51) Int. Cl.: C07H 9/04

(54) **Verfahren zur Herstellung von Isopropylidenderivaten von Sacchariden**
Method for the preparation of Isopropylidene derivatives of saccharides
Procédé de préparation de dérivés Isopropylidène de saccharides

(30) Priorität: 10.02.1993 DE 4303821
(43) Veröffentlichungstag der Anmeldung: 14.09.1994
(73) Patentinhaber: Boehringer Ingelheim Pharma KG, 55216 Ingelheim am Rhein (DE); BOEHRINGER INGELHEIM INTERNATIONAL GmbH, 55218 Ingelheim am Rhein (DE)
(72) Erfinder: Sobotta, Rainer, Dr. Dipl.-Chem., D-55218 Ingelheim/Rhein (DE); Klingler, Franz Dietrich, Dr. Dr.-Ing., D-64347 Griesheim/Hessen (DE)
(74) Vertreter: Laudien, Dieter, Dr.

(56) Entgegenhaltungen:
- EP-A- 0 076 118
- EP-A- 0 091 223
- EP-A- 0 139 486
- EP-A- 0 191 464

## Beschreibung

Die vorliegende Erfindung betrifft ein verbessertes Verfahren zur Herstellung von Isopropylidenderivaten von Sacchariden, insbesondere von 1,2-5,6-Diaceton-D-glucose (1,2:5,6-Di-O-isopropyliden-α-D-glucofuranose) 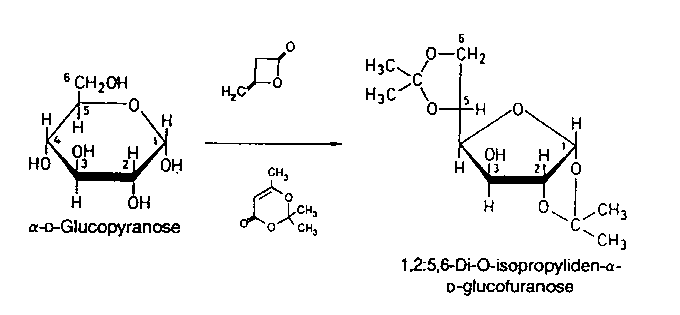 aus D-Glucose und Diketen oder dem Addukt des Diketens mit Aceton - mit der Bezeichnung 2,2,6-Trimethyl-1,3-dioxin-4-on.

1,2-5,6-Diaceton-D-glucose stellt unter den Isopropylidensaccariden ein zentrales Zwischenprodukt für zahlreiche andere Glucoseabkömmlinge dar, die u.a. als Arzneimittel große Bedeutung haben. Als Beispiele seien lediglich das 2-Deoxy-D-riboseanilid oder Amiprilose genannt.

Daneben kann 1,2-5,6-Diaceton-D-glucose als chiraler Ligand in Komplexen eingesetzt werden, die enantioselektive Reaktionen ermöglichen [F.D. Klingler und M. Psiorz, Chimicaoggi 1992, 47].

Diese zentrale Rolle der 1,2-5,6-Diaceton-D-glucose ist dafür verantwortlich, daß ein jährlicher Bedarf in Tonnengrößenordnungen an diesem Zwischenprodukt besteht.

Aus dem Stand der Technik ist allgemein bekannt, daß Monosacharide, die zwei sterisch benachbarte, cis-ständige OH-Gruppen enthalten, mit Aldehyden oder Ketonen in Gegenwart von Schwefelsäure, Zinkchlorid oder Phosphor(V)-oxid zu den entsprechenden Acetalen umgesetzt werden können (E. Fischer, 1895).

So ist die 1,2:5,6-Di-O-isopropyliden-α-D-glucofuranose (Diaceton-α-D-glucose) durch Umsetzung von D-Glucose mit Aceton in Gegenwart von Schwefelsäure erhältlich. Dabei muß - zur Erzielung hoher Umsätze - das aus der Ketalisierung resultierende Wasser gebunden bzw. aus dem Reaktionsgemisch entfernt werden.

Des weiteren ist aus dem Stand der Technik die Herstellung von Diacetonglucose in Gegenwart sauer reagierender Katalysatoren, wie Lewis-Säuren, beispielsweise komplexe Verbindungen des Bortrifluorids, Aluminiumhalogenide - wie zum Beispiel Aluminium(III)-chlorid oder -bromid -, Zinnsalze oder Halogenide seltener Erden bekannt.

Als weitere Katalysatoren sind aus dem Stand der Technik u.a. auch Jod, Gips oder Molekularsiebe bekannt. Jedoch ist die Verwendung der bislang als geeignet bekannten Katalysatoren - nicht nur hinsichtlich der Reaktionen im industriellen Maßstab - mit gravierenden Nachteilen verbunden, von denen beispielhaft die folgenden genannt sein sollen:
- bei der Verwendung von Mineralsäuren oder Phosphorpentoxid müssen große Mengen dieser Agenzien eingesetzt werden, was zum einen nur einen geringen Durchsatz erlaubt und zum anderen große Entsorgungsprobleme der aus der anschließend notwendigen Neutralisation resultierenden Salze mit sich bringt;
- bei der Verwendung von Jod sind große Mengen Lösungsmittel erforderlich, die ebenfalls nur einen geringen Durchsatz erlauben;
- beim Einsatz eines zusätzlichen Lösungsmittels, welches in der Lage ist, ein Azeotrop mit Wasser zu bilden, wird eine weitere Vergrößerung des Volumens des Reaktorgefäßes erforderlich - zudem geht der Einsatz eines Schleppmittels - wie beispielsweise Pentan - mit einer Siedepunktserniedrigung einher, womit die Reaktionstemperatur limitiert wird und womit sich die Reaktionszeit entsprechend verlängert;
- der Einsatz fester Katalysatoren bereitet durch die ebenfalls stattfindenden Karamelisierungsreaktionen ebenfalls Schwierigkeiten - außerdem ist beispielsweise die Wiederaufbereitung von Ionenaustauschern mit einem sehr großen Aufwand verbunden;
- daneben haftet vielen Umsetzungen der Nachteil an, daß durch die - in ihrem Ausmaß von den jeweiligen Reaktionsbedingungen abhängigen - Nebenreaktionen - wie z.B. die Selbstkondensation des Acetons - z.T. teerartige Nebenprodukte entstehen, die einerseits die Wirksamkeit des Katalysators beeinträchtigen und andererseits zu einer unerwünschten Verunreinigung des Reaktionsproduktes führen, die teilweise lediglich im Rahmen einer chromatographischen Reinigung wieder entfernbar sind.

Die Aufgabe der vorliegenden Erfindung besteht darin, die Nachteile der aus dem Stand der Technik bekannten Herstellverfahren für Diacetonglucose zu überwinden.

Erfindungsgemäß wird die Aufgabe dadurch gelöst, daß man D-Glucose mit Diketen oder mit dem Diketen-Aceton-Addukt (2,2,6-Trimethyl-1,3-dioxin-4-on) - welches als Diketen Äquivalent eingesetzt werden kann - in Aceton zur Reaktion bringt. Die Verwendung von Diketen bzw. des Diketen-Acetonadduktes bei der Herstellung von 1,2-5,6-Diaceton-D-glucose (1,2:5,6-Di-O-isopropyliden-α-D-glucofuranose) ist mit dem Vorteil verbunden, daß bei dieser Reaktion keine - u. U. schwierig - zu entsorgenden Abfallprodukte anfallen. Ferner kommt das erfindungsgemäße Verfahren mit einem relativ kleinen Lösungsmittelvolumen an Aceton aus.

Zur Durchführung des erfindungsgemäßen Verfahrens wird die - wasserfreie - D-Glucose und das Diketen oder das 2,2,6-Trimethyl-1,3-dioxin-4-on in Aceton gelöst und mit einer katalytischen Menge Lewis-Säure oder einer Brönsted-Säure versetzt. Als Lewis-Säuren finden bei dem erfindungsgemäßen Verfahren Halogenide des Bors, bevorzugt komplexe Borhalogenide und besonders bevorzugt der Bortrifluorid-Etherat-Komplex Verwendung. Als Brönstedt-Säure können Mineralsäuren - wie z.B. Schwefelsäure- oder organische Säuren - wie z.B. Toluolsulfonsäure - eingesetzt werden.

Das Reaktionsgemisch wird dann auf eine Temperatur im Bereich von 60 bis 120° C, bevorzugt 80 bis 100° C und besonders bevorzugt auf 90° C erhitzt. Nach dem Reaktionsende wird das Reaktionsgemisch abgekühlt und filtriert. Im anschließenden Reaktionsschritt wird das so erhaltene Filtrat mit der wässerigen Lösung einer alkalisch reagierenden Verbindung - bevorzugt mit der wässerigen Lösung eines Alkali- oder Erdalkalihydroxids und besonders bevorzugt mit verdünnter Natronlauge versetzt, bis das Reaktionsgemisch einen pH-Wert im Bereich von 6 bis 8, bevorzugt 6,5 bis 7,5 und besonders bevorzugt einen pH-Wert von ca. 7 erreicht hat. Danach wird das Aceton im Vakuum zum größten Teil abdestilliert und der Rückstand anschließend mit einem nicht mit Wasser mischbaren Extraktionsmittel extrahiert. Als Extraktionsmittel eignen sich hierzu aliphatische oder aromatische Kohlenwasserstoffe, bevorzugt halogenierte Kohlenwasserstoffe, Cycloalkane oder Alkylaromaten, worunter Dichlormethan, Cyclohexan und Toluol besonders bevorzugt werden. Die vereinigten Extrakte werden - gegebenenfals nach dem Trocknen - im Vakuum eingeengt und der verbliebene Rückstand aus einem geeigneten organischen Lösungsmittel umkristallisiert. Hierzu eignen sich Alkane - wie Erdölfraktionen - und insbesonderee Cycloalkane - wie zum Beispiel Cyclohexan. Anschließend wird das so erhaltene Kristallisat isoliert und getrocknet.

Die eingangs genannten Aufgaben werden durch die in den Beispielen beschriebenen Verfahren gelöst. Verschiedenartige, andere Ausgestaltungen des Verfahrens werden für den Fachmann aus der vorliegenden Beschreibung ersichtlich. Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind. Insbesondere wird darauf hingewiesen, daß die in den Beispielen zur Herstellung von 1,2:5,6-Di-O-isopropyliden-α-D-glucofuranose beschriebene Synthesefolge auf die Herstellung anderer Isopropyliden-Zucker übertragen werden kann.

Als in Frage kommende Ausgangsstoffe seien beispielsweise folgende Monosaccharide genannt:
D-Galactose, L- sowie D-Arabinose, Fructose, Sorbose, D-Xylose, D-Mannose, D-Ribose, D-Mannit oder L-Ascorbinsäure.

### Beispiel 1

### Umsetzung von D-glucose mit Diketen

54,1 g (300 mmol) wasserfreie α-D-(+)-Glucose und 26,5 g (300 mmol) Diketen (3-Hydroxy-3-butensäure-β-lacton) werden in 1,1 l Aceton gelöst und mit 0,85 g (5,2 mmol) Bortrifluorid-Diethylether-Komplex versetzt und in einem Rührautoklaven unter Rühren über einen Zeitraum von ca. 4,5 h auf 90°C erhitzt. Nach dem Abkühlen auf Raumtemperatur wird die Reaktionslösung filtriert und mit 350 ml 1 %-iger Natronlauge versetzt. Anschließend wird das Aceton in Vakuum abdestilliert. Der verbliebene Rückstand wird dreimal mit Dichlormethan extrahiert. Die vereinigten Extrakte werden in Vakuum eingedampft und der verbliebene Rückstand wird aus Cyclohexan umkristallisiert. Man erhält die 1,2-5,6-Diaceton-D-glucose (1,2:5,6-Di-O-isopropyliden-α-D-glucofuranose) in Form eines farblosen kristallinen Festkörpers in einer Ausbeute von 63 % d. Th..

### Beispiel 2

### Umsetzung von D-Glucose mit dem Diketen-Acetonaddukt

54,1 g (300 mmol) wasserfreie α-D-(+)-Glucose und 50.2 g (300 mmol) Diketen-Acetonaddukt (2,2,6-Trimethyl-1,3-dioxin-4-on) (85 %ig) werden in 1.1 l Aceton gelöst und mit 0,85 g (5,2 mmol) Bortrifluorid-Diethylether-Komplex versetzt und in einem Rührautoklaven unter Rühren über einen Zeitraum von ca. 4,5 h auf 90°C erhitzt. Nach dem Abkühlen auf Raumtemperatur wird die Reaktionslösung filtriert und mit 350 ml 1 %-iger Natronlauge versetzt.

Anschließend wird das Aceton in Vakuum abdestilliert. Der verbliebene Rückstand wird dreimal mit Dichlormethan extrahiert. Die vereinigten Extrakte werden in Vakuum eingedampft und der verbliebene Rückstand wird aus Cyclohexan umkristallisiert. Man erhält die 1,2-5,6-Diaceton-D-glucose (1,2:5,6-Di-O-isopropyliden-α-D-glucofuranose) in Form eines farblosen kristallinen Festkörpers in einer Ausbeute von 58 % d. Th..

## Patentansprüche

1. Verfahren zur Herstellung von Isopropylidenderivaten von Sacchariden, die zwei cis-ständige benachbarte Hydroxylgruppen besitzen, dadurch gekennzeichnet, daß man das Mono-, Di- oder Polysaccharid mit Diketen oder mit dem Additionsprodukt von Diketen mit Aceton umsetzt.

2. Verfahren zur Herstellung von Isopropylidenderivaten nach Anspruch 1, dadurch gekennzeichnet, daß das Saccharid D-Glucose, D-Galactose, L-Arabinose, D-Arabinose, Fructose, Sorbose, D-Xylose, D-Mannose, D-Ribose, D-Mannitol ist oder L-Ascorbinsäure eingesetzt wird.

3. Verfahren nach Anspruch 1 oder 2, dadurch gekennzeichnet, daß als Reaktionsmedium Aceton eingesetzt wird.

4. Verfahren zur Herstellung von 1,2-5,6-Diaceton-D-glucose nach einem der Ansprüche 1 bis 3, dadurch gekennzeichnet, daß man α-D-Glucose mit Diketen in Gegenwart einer Lewis-Säure oder einer Brönsted-Säure in Aceton bei einer Temperatur im Bereich von 60 bis 120° C zur Reaktion bringt, nach beendeter Reaktion die Reaktionsmischung abkühlt, von festen Bestandteilen befreit und anschließend mit der wässerigen Lösung einer alkalisch reagierenden Verbindung versetzt, bis ein pH-Wert im Bereich von 6 bis 8 erreicht ist, das Aceton abdestilliert, den Rückstnd mit einem mit Wasser nicht mischbaren organischen Extraktionsmittel extrahiert, die vereinigten Extrakte einengt, den Rückstand aus einem organischen Lösungsmittel umkristallisiert und die 1,2-5,6-Diaceton-D-glucose isoliert.

5. Verfahren nach Anspruch 4, dadurch gekennzeichnet, daß man α-D-Glucose mit Diketen in Gegenwart von Borhalogeniden oder komplexen Borhalogeniden oder in Gegenwart einer Mineralsäure oder einer organischen Säure in Aceton bei einer Temperatur im Bereich von 80 bis 100° C zur Reaktion bringt, nach beendeter Reaktion die Reaktionsmischung abkühlt von festen Bestandteilen befreit und anschließend mit der wässerigen Lösung eines Alkali- oder Erdalkalihydroxids versetzt, bis ein pH-Wert im Bereich von 6,5 bis 7,5 erreicht ist, das Aceton abdestilliert, den Rückstand mit einem halogenierten Kohlenwasserstoff, einem Cycloalkan oder einem Alkylaromaten extrahiert, die vereinigten Extrakte einengt, den Rückstand aus einem Alkan oder einem Cycloalkan umkristallisiert und die 1,2-5,6-Diaceton-D-glucose isoliert.

6. Verfahren nach Anspruch 4, dadurch gekennzeichnet, daß man α-D-Glucose mit Diketen in Gegenwart des Bortrifluorid-Diethylether-Komplexes bei einer Temperatur von 90° C zur Reaktion bringt, nach beendeter Reaktion die Reaktionsmischung abkühlt, filtriert und anschließend mit verdünnter Natronlauge versetzt bis ein pH-Wert von 7 erreicht ist, das Aceton abdestilliert, den Rückstand mit Dichlormethan extrahiert, die vereinigten Extrakte einengt, den Rückstand aus Cyclohexan umkristallisiert und die 1,2-5,6-Diaceton-D-glucose isoliert.

7. Verfahren zur Herstellung von 1,2-5,6-Diaceton-D-glucose nach einem der Ansprüche 1 bis 3, dadurch gekennzeichnet, daß man α-D-Glucose mit 2,2,6-Trimethyl-1,3-dioxin-4-on in Gegenwart einer Lewis-Säure oder einer Brönsted-Säure in Aceton bei einer Temperatur im Bereich von 60 bis 120° C zur Reaktion bringt, nach beendeter Reaktion die Reaktionsmischung abkühlt, von festen Bestandteilen befreit und anschließend mit der wässerigen Lösung einer alkalisch reagierenden Verbindung versetzt, bis ein pH-Wert im Bereich von 6 bis 8 erreicht ist, das Aceton abdestilliert, den Rückstnd mit einem mit Wasser nicht mischbaren organischen Extraktionsmittel extrahiert, die vereinigten Extrakte einengt, den Rückstand aus einem organischen Lösungsmittel umkristallisiert und die 1,2-5,6-Diaceton-D-glucose isoliert.

8. Verfahren nach Anspruch 7, dadurch gekennzeichnet, daß man α-D-Glucose mit 2,2,6-Trimethyl-1,3-dioxin-4-on in Gegenwart von Borhalogeniden oder komplexen Borhalogeniden oder in Gegenwart einer Mineralsäure oder einer organischen Säure in Aceton bei einer Temperatur im Bereich von 80 bis 100° C zur Reaktion bringt, nach beendeter Reaktion die Reaktionsmischung abkühlt von festen Bestandteilen befreit und anschließend mit der wässerigen Lösung eines Alkali- oder Erdalkalihydroxids versetzt, bis ein pH-Wert im Bereich von 6,5 bis 7,5 erreicht ist, das Aceton abdestilliert, den Rückstand mit einem halogenierten Kohlenwasserstoff, einem Cycloalkan oder einem Alkylaromaten extrahiert, die vereinigten Extrakte einengt, den Rückstand aus einem Alkan oder einem Cycloalkan umkristallisiert und die 1,2-5,6-Diaceton-D-glucose isoliert.

9. Verfahren nach Anspruch 7, dadurch gekennzeichnet, daß man α-D-Glucose mit 2,2,6-Trimethyl-1,3-dioxin-4-on in Gegenwart des Bortrifluorid-Diethylether-Komplexes bei einer Temperatur von 90° C zur Reaktion bringt, nach beendeter Reaktion die Reaktionsmischung abkühlt, filtriert und anschließend mit verdünnter Natronlauge versetzt bis ein pH-Wert von 7 erreicht ist, das Aceton abdestilliert, den Rückstand mit Dichlormethan extrahiert, die vereinigten Extrakte einengt, den Rückstand aus Cyclohexan umkristallisiert und die 1,2-5,6-Diaceton-D-glucose isoliert.

## Claims

1. Process for preparing isopropylidene derivatives of saccharides which have two adjacent hydroxyl groups in the cis position, characterised in that the mono-, di- or polysaccharide is reacted with diketene or with the addition product of diketene with acetone.

2. Process for preparing isopropylidene derivatives according to claim 1, characterised in that the saccharide is D-glucose, D-galactose, L-arabinose, D-arabinose, fructose, sorbose, D-xylose, D-mannose, D-ribose or D-mannitol, or that L-ascorbic acid is used.

3. Process according to claim 1 or 2, characterised in that acetone is used as the reaction medium.

4. Process for preparing 1,2-5,6-diacetone-D-glucose according to one of claims 1 to 3, characterised in that α-D-glucose is reacted with diketene in the presence of a Lewis acid or a Brönsted acid in acetone at a temperature in the range from 60 to 120°C, at the end of the reaction the mixture is cooled, freed from any solid constituents and then combined with the aqueous solution of an alkaline-reacting compound, until a pH in the range from 6 to 8 is achieved, the acetone is distilled off, the residue is extracted with a water-immiscible organic extraction agent, the combined extracts are evaporated down, the residue is recrystallised from an organic solvent and the 1,2-5,6-diacetone-D-glucose is isolated.

5. Process according to claim 4, characterised in that α-D-glucose is reacted with diketene in the presence of boron halides or complex boron halides or in the presence of an inorganic acid or an organic acid in acetone at a temperature in the range from 80 to 100°C, after the reaction has ended the reaction mixture is cooled, freed from solid constituents and then combined with the aqueous solution of an alkaline or alkaline earth metal hydroxide until a pH in the range from 6.5 to 7.5 is achieved, the acetone is distilled off, the residue is extracted with a halogenated hydrocarbon, a cycloalkane or an alkyl aromatic, the combined extracts are evaporated down, the residue is recrystallised from an alkane or a cycloalkane and the 1,2-5,6-diacetone-D-glucose is isolated.

6. Process according to claim 4, characterised in that α-D-glucose is reacted with diketene in the presence of the boron trifluoride/diethylether complex at a temperature of 90°C, after the reaction has ended the reaction mixture is cooled, filtered and then dilute sodium hydroxide solution is added until a pH of 7 is achieved, the acetone is distilled off, the residue is extracted with dichloromethane, the combined extracts are evaporated down, the residue is recrystallised from cyclohexane and the 1,2-5,6-diacetone-D-glucose is isolated.

7. Process for preparing 1,2-5,6-diacetone-D-glucose according to one of claims 1 to 3, characterised in that α-D-glucose is reacted with 2,2,6-trimethyl-1,3-dioxin-4-one in the presence of a Lewis acid or a Brönsted acid in acetone at a temperature in the range from 60 to 120°C, after the reaction has ended the reaction mixture is cooled, freed from solid constituents and then the aqueous solution of an alkaline-reacting compound is added until a pH in the range from 6 to 8 is achieved, the acetone is distilled off, the residue is extracted with a water-immiscible organic extraction agent, the combined extracts are evaporated down, the residue is recrystallised from an organic solvent and the 1,2-5,6-diacetone-D-glucose is isolated.

8. Process according to claim 7, characterised in that α-D-glucose is reacted with 2,2,6-trimethyl-1,3-dioxin-4-one in the presence of boron halides or complex boron halides or in the presence of an inorganic acid or an organic acid in acetone at a temperature in the range from 80 to 100°C, after the reaction has ended the reaction mixture is cooled, freed from solid constituents and then the aqueous solution of an alkaline or alkaline earth metal hydroxide is added until a pH in the range from 6.5 to 7.5 is achieved, the acetone is distilled off, the residue is extracted with a halogenated hydrocarbon, a cycloalkane or an alkyl aromatic, the combined extracts are evaporated down, the residue is recrystallised from an alkane or a cycloalkane and the 1,2-5,6-diacetone-D-glucose is isolated.

9. Process according to claim 7, characterised in that α-D-glucose is reacted with 2,2,6-trimethyl-1,3-dioxin-4-one in the presence of the boron trifluoridediethylether complex at a temperature of 90°C, after the reaction has ended the reaction mixture is cooled and filtered and then dilute sodium hydroxide solution is added until a pH of 7 is achieved, the acetone is distilled off, the residue is extracted with dichloromethane, the combined extracts are evaporated down, the residue is recrystallised from cyclohexane and the 1,2-5,6-diacetone-D-glucose is isolated.

## Revendications

1. Procédé de préparation de dérivés isopropylidène de saccharides qui possèdent deux groupes hydroxyle en cis voisins caractérisé en ce que l'on fait réagir le mono-, di- ou polysaccharide avec le dicétène ou avec le produit d'addition de dicétène avec l'acétone.

2. Procédé de préparation de dérivés isopropylidène selon la revendication 1 caractérisé en ce que le saccharide est le D-glucose, le D-galactose, le L-arabinose, le D-arabinose, le fructose, le sorbose, le D-xylose, le D-mannose, le D-ribose, le D-mannitol ou on utilise l'acide L-ascorbique.

3. Procédé selon la revendication 1 ou 2 caractérisé en ce que l'on utilise l'acétone comme milieu réactionnel.

4. Procédé de préparation du 1,2-5,6-diacétone-D-glucose selon l'une des revendications 1 à 3 caractérisé en ce que l'on fait réagir l'α-D-glucose avec le dicétène en présence d'un acide de Lewis ou d'un acide de Brönsted dans l'acétone à une température dans le domaine de 60 à 120°C, après la fin de la réaction on refroidit le mélange réactionnel, on le débarrasse des constituants solides puis on ajoute la solution aqueuse d'un composé à réaction alcaline, jusqu'à ce qu'un pH dans le domaine de 6 à 8 soit atteint, on élimine l'acétone par distillation, on extrait le résidu avec un agent d'extraction organique non miscible à l'eau, on concentre les extraits réunis, on recristallise le résidu dans un solvant organique et on isole le 1,2-5,6-diacétone-D-glucose.

5. Procédé selon la revendications 4 caractérisé en ce que l'on fait réagir l'α-D-glucose avec le dicétène en présence d'halogénures de bore ou d'halogénures de bore complexes ou en présence d'un acide minéral ou d'un acide organique dans l'acétone à une température dans le domaine de 80 à 100°C, après la fin de la réaction on refroidit le mélange réactionnel, on le débarrasse des constituants solides puis on ajoute la solution aqueuse d'un hydroxyde alcalin ou alcalino-terreux, jusqu'à ce qu'un pH dans le domaine de 6,5 à 7,5 soit atteint, on élimine l'acétone par distillation, on extrait le résidu avec un hydrocarbure halogéné, un cycloalcane ou un composé alkylaromatique, on concentre les extraits réunis, on recristallise le résidu dans un alcane ou un cycloalcane et on isole le 1,2-5,6-diacétone-D-glucose.

6. Procédé selon la revendications 4 caractérisé en ce que l'on fait réagir l'α-D-glucose avec le dicétène en présence du complexe trifluorure de bore-diéthyléther à une température de 90°C, après la fin de la réaction on refroidit le mélange réactionnel, on le filtre puis on ajoute de la lessive de soude diluée jusqu'à ce qu'un pH de 7 soit atteint, on élimine l'acétone par distillation, on extrait le résidu avec du dichlorométhane, on concentre les extraits réunis, on recristallise le résidu dans le cyclohexane et on isole le 1,2-5,6-diacétone-D-glucose.

7. Procédé de préparation du 1,2-5,6-diacétone-D-glucose selon l'une des revendications 1 à 3 caractérisé en ce que l'on fait réagir l'α-D-glucose avec la 2,2,6-triméthyl-1,3-dioxin-4-one en présence d'un acide de Lewis ou d'un acide de Brönsted dans l'acétone à une température dans le domaine de 60 à 120°C, après la fin de la réaction on refroidit le mélange réactionnel, on le débarrasse des constituants solides puis on ajoute la solution aqueuse d'un composé à réaction alcaline, jusqu'à ce qu'un pH dans le domaine de 6 à 8 soit atteint, on élimine l'acétone par distillation, on extrait le résidu avec un agent d'extraction organique non miscible à l'eau, on concentre les extraits réunis, on recristallise le résidu dans un solvant organique et on isole le 1,2-5,6-diacétone-D-glucose.

8. Procédé selon la revendications 7 caractérisé en ce que l'on fait réagir l'α-D-glucose avec la 2,2,6-triméthyl-1,3-dioxin-4-one en présence d'halogénures de bore ou d'halogénures de bore complexes ou en présence d'un acide minéral ou d'un acide organique dans l'acétone à une température dans le domaine de 80 à 100°C, après la fin de la réaction on refroidit le mélange réactionnel, on le débarrasse des constituants solides puis on ajoute la solution aqueuse d'un hydroxyde alcalin ou alcalino-terreux, jusqu'à ce qu'un pH dans le domaine de 6,5 à 7,5 soit atteint, on élimine l'acétone par distillation, on extrait le résidu avec un hydrocarbure halogéné, un cycloalcane ou un composé alkylaromatique, on concentre les extraits réunis, on recristallise le résidu dans un alcane ou un cycloalcane et on isole le 1,2-5,6-diacétone-D-glucose.

9. Procédé selon la revendications 7 caractérisé en ce que l'on fait réagir l'α-D-glucose avec la 2,2,6-triméthyl1,3-dioxin-4-one en présence du complexe trifluorure de bore-diéthyléther à une température de 90°C, après la fin de la réaction on refroidit le mélange réactionnel, on le filtre puis on ajoute de la lessive de soude diluée jusqu'à ce qu'un pH de 7 soit atteint, on élimine l'acétone par distillation, on extrait le résidu avec du dichlorométhane, on concentre les extraits réunis, on recristallise le résidu dans le cyclohexane et on isole le 1,2-5,6-diacétone-D-glucose.
